# DEMANDE DE BREVET EUROPEEN

(11) **EP 4 005 547 A1**
(43) Date de publication de la demande: **01.06.2022**
(21) Numéro de dépôt: 21216412.3
(22) Date de dépôt: 12.07.2017
(51) Int. Cl.: A61J 1/10, A61J 1/14

(54) **RECIPIENT BIOPHARMACEUTIQUE, POCHE DE RECIPIENT BIOPHARMACEUTIQUE, PROCEDE DE FABRICATION ET D'UTILISATION DE RECIPIENT BIOPHARMACEUTIQUE**

(30) Priorité: 22.07.2016 FR 1670339
(62) Demande divisionnaire de: 17751789.3
(71) Demandeur: SARTORIUS STEDIM FMT SAS, 13400 Aubagne (FR)
(72) Inventeur: Mendyk, Nicolas, 84220 Cabrières-d'Avignon (FR)

(57) **Abrégé**

L'invention concerne un récipient biopharmaceutique, comprenant : une poche (1) destinée à être fixée sur un connecteur de récipient (31), une trompe (10) flexible retroussée dans la poche (1) et déployable à l'extérieur de la poche (1) caractérisé en ce que la trompe (10) est un col (4) de taille réduite prolongeant le corps (2) de la poche (1), ledit col (4) comprenant une marque visuelle (34) représentative de la position limite de retroussage du col (4) à l'intérieur du corps (2) de la poche (1).

## Description

### DOMAINE DE L'INVENTION

L'invention concerne le domaine des récipients biopharmaceutiques, des poches de récipients biopharmaceutiques, ainsi que des procédés de fabrication et d'utilisation des récipients biopharmaceutiques. L'invention peut également être appliquée aux étapes de remplissage final de poches biopharmaceutiques sous conditions aseptiques, y compris pour un remplissage de fluide biopharmaceutique comme décrit par exemple dans le brevet EP 2534052B1 incorporé par référence.

### CONTEXTE DE L'INVENTION

Les récipients biopharmaceutiques sont remplis d'un contenu biopharmaceutique, notamment de type matériels biopharmaceutiques ou produits biopharmaceutiques solides ou liquides. Ce contenu biopharmaceutique, souvent stérile ou stérilisé, par exemple stérilisé par irradiations gamma ou par vapeur, va usuellement être transféré dans une chambre biopharmaceutique souvent stérile, par exemple un isolateur, pour y être stocké ou bien pour réaliser une étape de fabrication d'un produit biopharmaceutique ou pour procéder au remplissage final de ce produit. Le transfert entre récipient biopharmaceutique et chambre biopharmaceutique est une opération délicate au cours de laquelle le contenu biopharmaceutique peut être contaminé simplement par l'environnement extérieur souvent non stérile. La structure du récipient biopharmaceutique et la structure correspondante de la chambre biopharmaceutique sont déterminées d'une part pour éradiquer, minimiser ou au moins réduire ce risque de contamination, et d'autre part pour faciliter le transfert du contenu du récipient vers la chambre.

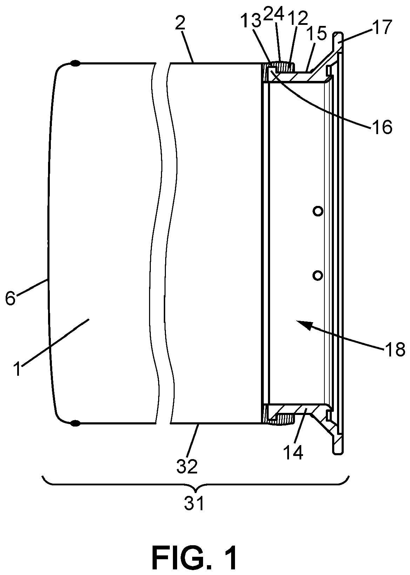

Il est connu un premier type de récipient biopharmaceutique, en particulier décrit en liaison avec la figure 1, dans lequel la poche du récipient est soudée sur le connecteur du récipient. En revanche, ce récipient pourrait être optimisé, à la fois par rapport à la sécurité du transfert et du risque de contamination associé ainsi que par rapport à la facilité et à la fluidité du transfert du contenu du récipient vers la chambre.

Selon l'invention, il s'agirait d'améliorer la protection de la zone critique, laquelle est souvent un anneau de passage entre récipient et chambre et qui est une zone sensible par laquelle des agents contaminants de l'extérieur pourraient s'introduire de manière privilégiée pour se retrouver dans l'espace de communication entre récipient et chambre, sur le passage du transfert du contenu à partir du récipient vers la chambre. Il s'agit également d'améliorer la fluidité du transfert du contenu à partir du récipient vers la chambre, en canalisant, voire en guidant ce transfert, surtout au niveau de l'espace de communication entre récipient et chambre.

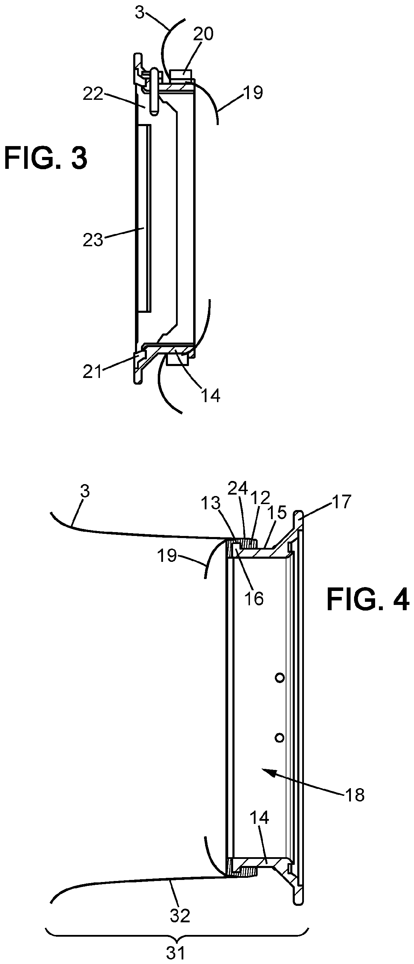

Il est connu un deuxième type de récipient biopharmaceutique, en particulier décrit en liaison avec la figure 3, dans lequel la poche du récipient est assemblée avec une trompe de protection de transfert par frettage, c'est-à-dire à l'aide d'une bague de frettage. Ce récipient présente l'intérêt, par rapport au premier type de récipient biopharmaceutique, à la fois d'une amélioration de la sécurité du transfert et d'une diminution du risque de contamination associé, ainsi qu'une amélioration de la facilité et de la fluidité du transfert du contenu du récipient vers la chambre. En revanche, ce récipient souffre d'une certaine complexité de structure et de fabrication.

Selon l'invention, il s'agirait de simplifier la structure et la fabrication du récipient, tout en lui permettant de continuer à assurer d'une part la protection de la zone critique de passage entre récipient et chambre par laquelle des agents contaminants de l'extérieur pourraient s'introduire dans l'espace de communication entre récipient et chambre, contaminant ou risquant de contaminer le contenu lors de son transfert, et d'autre part la fluidité du transfert du contenu à partir du récipient vers la chambre, en canalisant ou en guidant ce transfert en particulier dans l'espace de communication entre récipient et chambre.

### RESUME DE L'INVENTION

Le but de la présente invention est de fournir un récipient biopharmaceutique palliant au moins partiellement les inconvénients précités.

Plus particulièrement, l'invention vise à fournir un récipient biopharmaceutique présentant une simplification de structure et de fabrication par rapport au deuxième type de récipient, tout en présentant une amélioration de sécurité de transfert par rapport au risque de contamination et une amélioration de facilité de transfert par rapport au premier type de récipient.

Préférentiellement, l'invention vise à fournir un récipient biopharmaceutique présentant une simplification de structure et de fabrication comparables à celles du premier type de récipient, tout en présentant une amélioration de sécurité de transfert par rapport au risque de contamination et une amélioration de facilité de transfert comparables à celles du deuxième type de récipient.

Préférentiellement, l'invention propose de continuer à assurer d'une part la protection de la zone critique de passage entre récipient et chambre, comportant un risque de contamination particulier, et d'autre part la fluidité du transfert du contenu à partir du récipient vers la chambre par canalisation et/ou guidage de ce transfert, tout en simplifiant la structure et la fabrication du récipient, d'une part en diminuant le nombre de composants nécessaires à la réalisation du récipient et/ou d'autre part en diminuant le nombre d'étapes de fabrication du récipient, le tout éventuellement en facilitant aussi l'utilisation du récipient pour la réalisation du transfert de son contenu.

La simplification de la structure et/ou de la fabrication du récipient, tout en lui conservant des propriétés de sécurité contre le risque de contamination et/ou de facilitation du transfert de son contenu, qui soient identiques, similaires ou comparables, entraîne à qualité égale ou comparable, une diminution substantielle de son coût.

A cette fin, la présente invention propose un récipient biopharmaceutique, comprenant : un connecteur destiné à se fixer sur un connecteur de chambre, une poche, à fonction de contenant, dont une partie est fixée au connecteur, une trompe flexible retroussée dans la poche et déployable à l'extérieur de la poche au travers du connecteur, caractérisé en ce que : la trompe fait partie intégrante de la poche, la trompe est un col de taille réduite prolongeant le corps de la poche, ladite partie de poche fixée au connecteur est un repli de la paroi de la poche, la paroi de la poche forme d'une part le corps de la poche d'un côté du repli et d'autre part la trompe de l'autre côté du repli. Le connecteur considéré, sans plus de précision, dans le récipient biopharmaceutique juste décrit est bien sûr le connecteur du récipient, sauf lorsqu'il est mentionné explicitement que c'est un connecteur de chambre.

Ce récipient est un produit pratiquement finalisé au cours du procédé de fabrication du récipient.

La trompe fait partie intégrante de la poche. La trompe et la poche sont dans le même matériau, ou au moins une partie de la trompe et une partie de la poche sont dans le même matériau. La trompe et la poche ne sont qu'un seul élément, fabriqué d'un seul tenant, et non pas deux éléments fabriqués séparément puis assemblés entre eux.

Préférentiellement, la partie de la poche fixée au connecteur est une partie de la poche fixée autour du connecteur ; cette partie de la poche fixée au connecteur peut aussi être une partie de la poche fixée à l'intérieur du connecteur, ou encore au bord du connecteur.

Selon des modes de réalisation préférentiels, la trompe flexible a été directement intégrée dans la conception de la paroi de la poche. La trompe flexible et le reste du corps de la poche sont alors une seule et même pièce constituant la paroi de la poche, s'étendant du fond de la poche jusqu'à l'extrémité ouverte de la poche.

A cette fin, la présente invention propose aussi une poche de récipient biopharmaceutique, comprenant : un corps de poche à fonction de contenant, une partie de la poche destinée à être fixée à un connecteur du récipient, caractérisée en ce qu'elle comprend aussi : une trompe flexible, qui est retroussée dans le corps de la poche et déployable à l'extérieur du corps de la poche au travers de la partie de la poche destinée à être fixée à un connecteur du récipient, qui fait partie intégrante de la poche, et qui est un col de taille réduite prolongeant le corps de la poche, et en ce que : la partie de la poche destinée à être fixée à un connecteur du récipient est un repli de la paroi de la poche, la paroi de la poche forme d'une part le corps de la poche d'un côté du repli et d'autre part la trompe de l'autre côté du repli.

Cette poche est un produit intermédiaire au cours du procédé de fabrication du récipient.

A cette fin, la présente invention propose également une poche de récipient biopharmaceutique, comprenant : un corps de poche à fonction de contenant, caractérisée en ce qu'elle comprend aussi : un col de taille réduite, qui prolonge le corps de la poche, qui est destinée à être une trompe flexible de protection du transfert du contenu de la poche, et qui fait partie intégrante de la poche.

Cette poche est un produit initial au cours du procédé de fabrication du récipient.

A cette fin, la présente invention propose encore un procédé d'utilisation d'un récipient biopharmaceutique selon l'invention, caractérisé en ce qu'il comporte : une étape de fixation du récipient contre une ouverture d'une chambre, une étape de déploiement de la trompe flexible hors du corps de la poche au travers du connecteur et au travers de l'ouverture de la chambre, une étape de transfert du contenu du récipient vers la chambre au travers de la trompe déployée. Préférentiellement, entre l'étape de fixation et l'étape de déploiement, est réalisée une étape d'ouverture concomitante d'une part des portes du récipient biopharmaceutique et d'autre part des portes de la chambre.

De préférence, entre l'étape de fixation et l'étape de déploiement, une étape d'ouverture du connecteur de la poche et de la chambre, permettant une mise en communication des espaces intérieurs stériles de la poche et de la chambre, après l'étape de transfert, successivement une étape d'escamotage de la trompe flexible, une étape de fermeture du connecteur de la poche et de la chambre, et une étape de déconnexion du récipient et de la chambre.

A cette fin, la présente invention propose encore un procédé d'utilisation d'un récipient biopharmaceutique selon l'invention, caractérisé en ce qu'il comporte : une étape de fixation du récipient contre une ouverture d'une chambre, une étape de déploiement de la trompe flexible hors du corps de la poche au travers du connecteur et au travers de l'ouverture de la chambre, une étape de transfert du contenu usagé de la chambre vers le récipient au travers de la trompe déployée.

Ce procédé d'utilisation est un procédé d'utilisation d'un récipient selon l'invention.

A cette fin, la présente invention propose enfin un procédé de fabrication d'un récipient biopharmaceutique, comprenant : une étape de fabrication d'une poche comprenant un corps de poche prolongée par un col de taille réduite, une étape de retroussage du col dans le corps de la poche, formant un repli dans la paroi de la poche entre le corps de la poche et le col de la poche, une étape de fixation du repli à un connecteur destiné à être connecté à une chambre.

Ce procédé de fabrication est un procédé de fabrication d'un récipient selon l'invention.

Selon un autre objet de l'invention, il est également prévu un récipient biopharmaceutique, comprenant : un connecteur destiné à se fixer sur un connecteur de chambre, une poche, à fonction de contenant, dont une partie est fixée au connecteur, une trompe flexible retroussée dans la poche et déployable à l'extérieur de la poche au travers du connecteur, caractérisé en ce que : la trompe fait partie intégrante de la poche, la trompe prolonge le corps de la poche, la trompe présentant le même diamètre ou la même taille que le corps, ce même diamètre ou cette même taille correspondant à la plus grande dimension de la trompe dans une section étant compris entre 100mm et 300mm, de préférence compris entre 150mm et 250mm, encore plus de préférence compris entre 190mm et 210mm, ladite partie de poche fixée au connecteur est un repli de la paroi de la poche, la paroi de la poche forme d'une part le corps de la poche d'un côté du repli et d'autre part la trompe de l'autre côté du repli.

Pour des tailles de trompe élevées, correspondant à des tailles comparables de porte de chambre, le corps de la poche peut présenter une taille égale à celle de la trompe. De cette manière, le procédé de fabrication de la poche incluant un corps et une trompe de même taille est plus simple, puisque l'ensemble de la poche, corps et trompe, se résume à un sac de section de taille constante, sans rétrécissement. Ce type de poche, de section de taille constante, présente un intérêt particulier pour une certaine gamme de tailles correspondant à une certaine gamme de tailles de porte de chambre. La poche étant de préférence cylindrique à section droite circulaire, la gamme de diamètres correspondants correspond à 100-300mm, de préférence 150-250mm, encore plus de préférence 190-210mm, par exemple 200mm. La poche peut présenter une section droite différente d'une section droite circulaire, par exemple rectangulaire ou carrée.

Suivant des modes de réalisation préférés, l'invention comprend une ou plusieurs des caractéristiques suivantes qui peuvent être utilisées séparément ou en combinaison partielle entre elles ou en combinaison totale entre elles, avec l'un ou l'autre des objets de l'invention précédemment présentés.

De préférence, ladite partie de la poche fixée au connecteur est une partie de la poche fixée autour du connecteur.

Ainsi, le repli est plus solidement fixé au connecteur, en particulier lors du déploiement de la trompe au travers du connecteur, car lorsque la trompe se déploie en tirant sur le repli, elle a plutôt tendance à continuer à serrer le repli autour du connecteur, plutôt qu'à le décrocher vers l'intérieur du connecteur, ce qui pourrait être le cas si le repli était fixé sur le pourtour intérieur du connecteur.

Alternativement, ladite partie de la poche fixée au connecteur est une partie de la poche fixée sur le pourtour intérieur du connecteur.

De préférence, le repli de la paroi de la poche comprend deux portions de paroi de poche repliées l'une sur l'autre, sans espace libre entre elles, ces deux portions étant avantageusement fixées l'une contre l'autre, ces deux portions étant plus avantageusement soudées l'une contre l'autre.

Ainsi, cette zone sensible de raccord entre la paroi de la poche et le connecteur, non seulement ne se présente pas comme une zone de fragilité, mais encore se présente comme une zone de renforcement grâce aux deux couches bien solidarisées entre elles, couches qui sont déjà le repli d'une même paroi donc bien attachées l'une à l'autre et qui sont ainsi encore mieux attachées l'une à l'autre.

De préférence, le repli de la paroi de la poche est fixé sur le connecteur, un surmoulage étant disposé entre le connecteur et le repli, le repli étant avantageusement soudé sur le surmoulage.

Ainsi, le matériau du connecteur, plutôt un matériau rigide, n'a pas besoin de présenter de compatibilité de soudure avec le matériau de la poche, plutôt un matériau souple. Le surmoulage présentant une épaisseur notable peut être aisément surmoulé au connecteur, malgré des matériaux non compatibles en soudure. Le surmoulage sera choisi dans un matériau compatible, et même facilement compatible, en soudage, avec le matériau de la poche.

De préférence, le surmoulage comprend un creux dont la forme intérieure épouse la forme extérieure d'une protubérance de la paroi extérieure du connecteur.

Ainsi la solidité de l'attachement entre le surmoulage d'une part et le connecteur d'autre part est augmentée.

De préférence, l'intérieur du récipient est stérile.

Cela rend l'invention d'autant plus intéressante que le risque de contamination lors du transfert du contenu du récipient vers la chambre est critique et doit absolument être évité.

De préférence, la poche est en plastique souple, de préférence en plastique non élastique. Le matériau utilisé est par exemple un matériau thermoplastique qui sera utilisé en épaisseur suffisamment fine pour donner la souplesse requise.

Dans une alternative, la poche peut être en plastique souple et relativement élastique pour préserver l'intégrité du récipient.

Ainsi, la poche est facile à fabriquer et à utiliser, en particulier lors de son remplissage par un contenu et lors du transfert de son contenu. La poche évite préférentiellement les risques de fragilisation liés à une déformation élastique excessive soit arrivant à rupture soit rendant le matériau plastique plus poreux aux agents contaminants.

De préférence, la poche est à symétrie de révolution ou la partie de la poche destinée à être soudée au connecteur est à symétrie de révolution.

Ainsi, la poche est plus facile à fabriquer et à utiliser.

Plusieurs types de designs de poches peuvent être envisagés. Dans un premier type de design, une poche en deux dimensions est extrudée. Dans un deuxième type de design, une poche en deux dimensions est assemblée. Dans un troisième type de design, une poche en trois dimensions est réalisée de forme générale parallélépipédique.

De préférence, le récipient est rempli de matériels biopharmaceutiques à transférer dans une chambre. Ce matériel biopharmaceutique peut être par exemple du matériel de conditionnement, des bouchons de seringue, des produits biopharmaceutiques, des médicaments, ou des outils stérilisés utilisés pour des opérations dans le domaine biopharmaceutique, ainsi que des conteneurs finaux, ou encore des produits à usage unique pour le transfert de fluide.

De préférence, le connecteur présente un diamètre compris entre 80mm et 300mm, de préférence entre 100mm et 210mm ou bien le connecteur présente une forme rectangulaire avec une longueur de diagonale comprise entre 100mm et 350mm.

Ainsi, la taille relativement faible de l'espace de communication entre récipient et chambre rend d'autant plus intéressante l'invention que celle-ci améliore la lutte contre le risque de contamination et la facilité du transfert du contenu du récipient vers la chambre, deux aspects d'autant plus sensibles voire d'autant plus critiques que cet espace de communication est exigu.

De préférence, dans la poche de récipient biopharmaceutique dans sa forme finalisée, la trompe n'est retroussée que sur une partie de sa longueur, avantageusement sur une partie comprise entre un tiers et deux tiers de sa longueur, encore plus avantageusement sur une partie valant environ la moitié de sa longueur.

De préférence, la trompe présente une longueur qui est comprise entre 100mm et 900mm, de préférence comprise entre 300mm et 900mm, et encore plus de préférence comprise entre 600mm et 900mm.

Ainsi, d'une part il est plus facile de ne retrousser la trompe que sur une partie de sa longueur lors de la fabrication du récipient, et d'autre part il est également plus facile de disposer d'une partie de la trompe non retroussée pour déployer plus facilement la trompe au travers du connecteur lors de l'utilisation du récipient.

Alternativement, la trompe peut ne pas être retroussée, mais être par exemple laissée à plat à l'intérieur du récipient ou encore être pliée en accordéon au niveau du connecteur.

De préférence, dans la poche de récipient biopharmaceutique, le col comprend une marque visuelle représentative de la position limite de retroussage du col à l'intérieur du corps de la poche. Cette marque visuelle sert de détrompeur permettant de visualiser la position d'arrêt souhaitée pour le retroussage. Ce détrompeur très simple simplifie et facilite encore le procédé de fabrication de la poche et donc du récipient.

De préférence, cette marque visuelle est la fin d'une longueur de soudure supplémentaire le long de la poche, le début de cette longueur de soudure supplémentaire le long de la poche coïncidant avec l'extrémité du col de la poche jouxtant le rétrécissement de la poche. Ainsi, cette marque visuelle présente les avantages suivants : d'abord, elle reste bien visible tout le temps, puis, elle peut être réalisée pendant les étapes de soudure lors du procédé de fabrication de la poche, et non pas lors d'une étape supplémentaire distincte du procédé de fabrication de la poche, ensuite elle ne nécessite pas d'encre ni de couleur toujours potentiellement susceptible de se diffuser au travers de la paroi de la poche et de contaminer le contenu stérile ou fragile de la poche.

Cet aspect pourrait aussi être utilisé seul, indépendamment de tout ou partie du reste du récipient selon l'invention. Un autre objet de l'invention est, dans ce cas, une poche de récipient biopharmaceutique, comprenant : un corps de poche à fonction de contenant, une partie de la poche destinée à être fixée à un connecteur du récipient, caractérisée en ce qu'elle comprend aussi : une trompe flexible, qui est retroussée dans le corps de la poche et déployable à l'extérieur du corps de la poche au travers de la partie de la poche destinée à être fixée à un connecteur du récipient, qui est un col de taille réduite prolongeant le corps de la poche, et en ce que : le col comprend une marque visuelle représentative de la position limite de retroussage du col à l'intérieur du corps de la poche, le col comprend au moins une soudure longitudinale délimitant le col en s'étendant tout le long du col, le col comprend au moins une soudure supplémentaire s'étendant le long d'une partie seulement de cette soudure longitudinale, l'extrémité de cette soudure supplémentaire, située du côté de l'ouverture du col, constituant ladite marque visuelle.

De préférence, la longueur de la soudure supplémentaire dont l'extrémité matérialise la marque visuelle correspond sensiblement à la largeur de l'anneau, circulaire ou non, constituant le connecteur.

De préférence, dans la poche de récipient biopharmaceutique, l'extrémité libre de la trompe ou du col est ouverte.

Ainsi, la trompe sera prête à être utilisée dès qu'elle aura été déployée, sans nécessiter d'étape supplémentaire d'ouverture d'un orifice en bout de trompe pour permettre le passage du contenu lors de son transfert du récipient vers la chambre. Par ailleurs, le fait que la trompe soit retroussée à l'intérieur de la poche et que le passage du connecteur soit fermé par un autre élément, permet au bout de trompe d'être ouvert sans inconvénient notable pour la stérilité du récipient.

De préférence, dans la poche de récipient biopharmaceutique, la poche comprend un rétrécissement progressif du corps de la poche avant la trompe ou le col de la poche.

Ce rétrécissement, avantageusement en entonnoir, et préférentiellement conique, augmente la fluidité du transfert du contenu du récipient vers la chambre.

De préférence, dans la poche de récipient biopharmaceutique, la trompe ou le col présente une section au moins 4 fois plus petite que la section du corps de la poche, avantageusement au moins 10 fois plus petite.

Ainsi, la fluidité du transfert du contenu du récipient vers la chambre reste garantie, même lorsque le récipient présente une section de corps importante, ce qui augmente sa contenance, et lorsque simultanément l'ouverture de la chambre présente une section relativement faible, ce qui diminue l'encombrement et la complexité de la porte fermant cette ouverture de la chambre en l'absence de transfert en provenance d'un récipient.

De préférence, dans la poche de récipient biopharmaceutique, la trompe ou le col présente une longueur valant au moins le dixième de la longueur du corps de la poche, avantageusement au moins le quart.

Ainsi, la trompe permet une canalisation et même un guidage au travers de tout l'espace de communication situé entre récipient et chambre, de manière à libérer le contenu du récipient seulement bien à l'intérieur de la chambre, là où il peut être plus facilement manipulé par un utilisateur ou par un robot.

De préférence, le procédé de fabrication d'un récipient biopharmaceutique comporte aussi : une étape de fermeture du connecteur par une porte de manière à rendre étanche le récipient au niveau du connecteur, une étape de remplissage du récipient par du contenu, une étape de fermeture du fond de poche de manière à rendre étanche le récipient au niveau du fond de poche, une étape de stérilisation du récipient rempli de contenu. Ainsi, la porte est fermée avant remplissage du récipient, et le fond de la poche reste ouvert : la poche est remplie, et seulement ensuite le fond de la poche sera alors fermé.

Alternativement, le procédé de fabrication d'un récipient biopharmaceutique comporte aussi : une étape de remplissage du récipient par du contenu, une étape de fermeture du connecteur par une porte de manière à rendre étanche le récipient au niveau du connecteur, une étape de stérilisation du récipient rempli de contenu.

Ainsi, un récipient rempli, fermé et stérile peut être facilement stocké et transporté, avant d'être amené au contact de la chambre pour y déverser son contenu stérile.

Selon une autre alternative, la poche comprend deux connecteurs, un à chaque extrémité, La poche est livrée stérile. Puis, une stérilisation de matériel est réalisée dans un autoclave muni d'un connecteur d'autoclave du type d'un connecteur de chambre ou d'isolateur. Ensuite, le matériel est transféré depuis l'autoclave dans la poche via le premier connecteur de la poche. Enfin, le deuxième connecteur de la poche sera utilisé pour réaliser la connexion entre la poche et l'isolateur.

De préférence, dans l'étape de fabrication, la poche est extrudée ou assemblée, entre l'étape retroussage et l'étape de fixation, une ou plusieurs étapes éventuelles de repliage.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES DESSINS

La figure 1 représente schématiquement une vue de coupe d'un exemple de premier type de récipient biopharmaceutique soudé sans trompe.
La figure 2 représente schématiquement une vue de coupe d'un exemple d'assemblage de chambre et de récipient biopharmaceutique utilisant un premier type de récipient biopharmaceutique soudé sans trompe.
La figure 3 représente schématiquement une vue de coupe d'un exemple de deuxième type de récipient biopharmaceutique fretté avec trompe selon l'art antérieur.
La figure 4 représente schématiquement une vue de coupe d'un exemple d'un troisième type récipient biopharmaceutique soudé avec trompe.
La figure 5 représente schématiquement une vue de coupe d'un exemple d'un mode de réalisation d'un récipient biopharmaceutique soudé avec trompe selon l'invention.
La figure 6 représente schématiquement un exemple d'une première étape d'un procédé de fabrication d'un mode de réalisation d'un récipient biopharmaceutique soudé avec trompe selon l'invention, ce récipient étant représenté de profil.
La figure 7 représente schématiquement un exemple d'une deuxième étape d'un procédé de fabrication d'un mode de réalisation d'un récipient biopharmaceutique soudé avec trompe selon l'invention, ce récipient étant représenté de profil.
La figure 8 représente schématiquement un exemple d'une troisième étape d'un procédé de fabrication d'un mode de réalisation d'un récipient biopharmaceutique soudé avec trompe selon l'invention, ce récipient étant représenté de profil.
La figure 9 représente schématiquement un exemple d'une première étape d'un procédé d'utilisation d'un mode de réalisation d'un récipient biopharmaceutique soudé avec trompe selon l'invention, ce récipient étant représenté de profil.
La figure 10 représente schématiquement un exemple d'une deuxième étape d'un procédé d'utilisation d'un mode de réalisation d'un récipient biopharmaceutique soudé avec trompe selon l'invention, ce récipient étant représenté de profil.
La figure 11 représente un premier exemple de type de procédé de fabrication d'une poche selon l'invention, par extrusion d'un film plastique.
La figure 12 représente un deuxième exemple de type de procédé de fabrication d'une poche selon l'invention, par soudage de deux feuilles de film plastique l'une sur l'autre.
La figure 13 représente un troisième exemple de type de procédé de fabrication d'une poche selon l'invention, par soudage de deux feuilles de film plastique l'une sur l'autre, avec soudage de deux replis latéraux entre les deux feuilles plastiques.
La figure 14 représente un exemple du fond d'une poche selon l'invention.
La figure 15 représente un exemple de poche selon l'invention, présentant une trompe courte.
La figure 16 représente un autre exemple de poche selon l'invention, présentant une trompe longue.
La figure 17 représente une étape du procédé de fabrication d'un exemple de poche selon l'invention, consistant essentiellement à retrousser le col dans la poche.
La figure 18 représente une étape du procédé de fabrication d'un exemple de poche selon l'invention, consistant essentiellement à remettre le bout du col dans la partie retroussée elle-même située dans la poche.
La figure 19 représente un exemple de poche selon l'invention, prête à être fixée sur un connecteur.
La figure 20 représente des exemples de dimensions d'une poche selon l'invention, correspondant aux premier et deuxième exemples de type de procédé de fabrication de la poche.
La figure 21 représente des exemples de dimensions d'une poche selon l'invention, correspondant au troisième exemple de type de procédé de fabrication de la poche.

### LISTE DES REFERENCES SUR LES FIGURES

- 1/: poche de récipient biopharmaceutique
- 2/: corps de poche de récipient biopharmaceutique
- 3/: rétrécissement de poche de récipient biopharmaceutique
- 4/: col de poche de récipient biopharmaceutique
- 5/: ouverture du col
- 6/: fond de poche de récipient biopharmaceutique
- 7/: repli de paroi de poche de récipient biopharmaceutique
- 8/: partie retroussée du col
- 9/: bout du col
- 10/: trompe de poche de récipient biopharmaceutique
- 11/: porte du récipient biopharmaceutique
- 12/: surmoulage
- 13/: creux du surmoulage
- 14/: connecteur du récipient biopharmaceutique
- 15/: surface de fixation du connecteur
- 16/: protubérance du connecteur
- 17/: rebord du connecteur
- 18/: espace de communication destiné à être fermé par la porte
- 19/: trompe séparée
- 20/: bague de frettage
- 21/: joint de porte de chambre
- 22/: couvercle de porte de récipient biopharmaceutique
- 23/: plaque métallique de porte de récipient biopharmaceutique
- 24/: pourtour de surmoulage
- 25/: porte de chambre
- 26/: bras de porte de chambre
- 27/: axe de rotation de bras de porte de chambre
- 28/: socle d'ancrage de l'axe de rotation
- 29/: paroi de la chambre
- 30/: chambre
- 31/: récipient biopharmaceutique
- 32/: paroi de la poche
- 33/: ouverture de la chambre
- 34/: marque visuelle
- 35/: soudure
- 36/: soudure supplémentaire de la marque visuelle
- 37: oreille
- 38/: trou
- 39/: codage couleur
- 40/: repli latéral de poche
- d/: diamètre de l'extrémité ouverte du col
- D/: diamètre de l'extrémité du rétrécissement fixée sur connecteur
- L/: longueur de trompe
- 1/: longueur de soudure supplémentaire
- α/: angle du rétrécissement
- L1/: longueur du corps de la poche
- L2/: largeur du corps de la poche
- h/: hauteur des replis latéraux de poche

### DESCRIPTION DETAILLEE DE L'INVENTION

La figure 1 représente schématiquement une vue de coupe d'un exemple de premier type de récipient biopharmaceutique soudé sans trompe.

Un récipient 31 comprend deux pièces principales qui sont une poche 1 souple mais non élastique et un connecteur 14 rigide, la poche 1 et le connecteur 14 étant tous deux cylindriques de révolution, la poche 1 étant fixée autour du connecteur 14 par l'intermédiaire d'un surmoulage 12 situé entre les deux. La poche 1, le connecteur 14 et le surmoulage 12, sont cylindriques à symétrie de révolution.

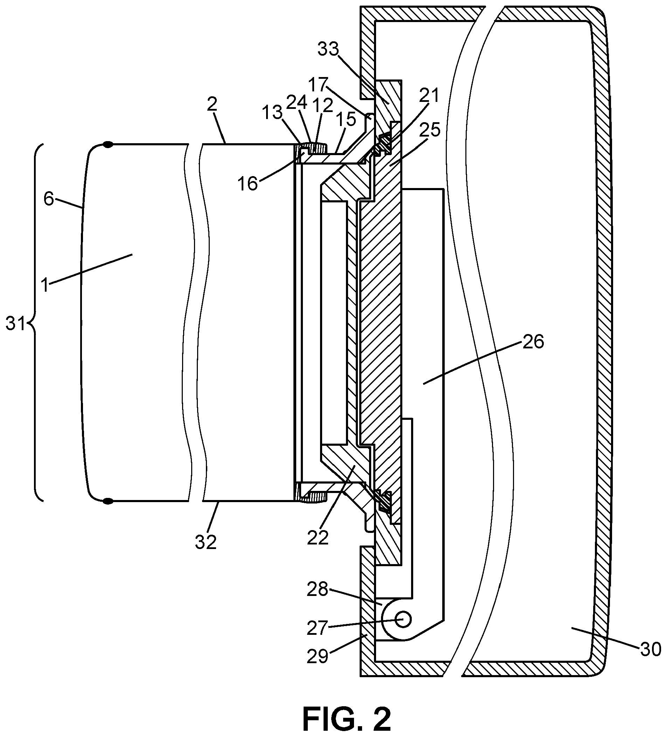

Le connecteur 14 comprend un rebord 17, une surface de fixation 15 présentant une protubérance 16. Au centre du connecteur 14 qui est annulaire se trouve l'espace de communication 18 situé entre récipient 31 et chambre non représentée sur cette figure 1 par souci de clarté mais représentée sur la figure 2.

Le surmoulage 12 comprend un pourtour 24 et un creux 13. Le surmoulage 12 est surmoulé de manière annulaire sur la surface de fixation 15 du connecteur 14, le creux 13 du surmoulage 12 s'emboîtant dans la protubérance 16 du connecteur 14.

La poche 1 comprend un fond 6, une paroi 32 cylindrique de révolution s'étendant le long d'un corps 2. Le corps 2 de la poche 1 est soudé de manière annulaire sur le pourtour 24 du surmoulage 12.

Il ne manque plus qu'un élément obturateur pour fermer l'espace de communication 18 pour que le récipient 31 soit finalisé. Soit le récipient 31 est rempli d'abord puis l'élément obturateur est mis en place ensuite, soit l'élément obturateur est mis en place d'abord, puis le récipient 31 est rempli ensuite. La poche 1 est fermée par son fond 6. Plus précisément, la poche 1 est par exemple tubulaire ou bien formée de deux feuilles de film apposées l'une sur l'autre dans le prolongement longitudinal l'une de l'autre. Pour la soudure du fond 6 de poche 1, l'extrémité de la poche 1 du côté du fond 6 est aplatie, et une soudure est réalisée en travers sur la largeur.

La figure 2 représente schématiquement une vue de coupe d'un exemple d'assemblage de chambre et de récipient biopharmaceutique utilisant un premier type de récipient biopharmaceutique soudé sans trompe.

Le récipient 31 de la figure 1 est représenté fixé sur l'ouverture 33 de la chambre 30, ou plus précisément sur le pourtour de l'ouverture 33 de la chambre 30. Cette ouverture 33 est fermée par une porte 25 de la chambre 30, un joint 21 étant disposé entre le pourtour de l'ouverture 33 et la porte 25. Cette ouverture 33, ou plus précisément ce pourtour de l'ouverture 33, joue le rôle de connecteur de chambre 30, et c'est sur ce connecteur 33 de chambre 30 qu'est fixé le connecteur 14 du récipient 31. Un couvercle 22 de porte du récipient 31 ferme ce récipient 31, et vient se fixer contre la porte 25 de la chambre 30. Un bras 26 est solidaire de la porte 25 pour faire basculer dans la chambre 30 l'ensemble solidarisé constitué par d'une part la porte du récipient 31 dont n'est représenté que le couvercle 22 et d'autre part la porte 25 de la chambre 30. Ce basculement est fait autour d'un axe de rotation 27 porté par un socle d'ancrage 28 à la paroi 29 de la chambre 30.

Lorsque le récipient 31 est fixé contre l'ouverture 33 de la chambre 30, l'ensemble solidarisé constitué par le couvercle 22 et la porte 25, porté par le bras 26, bascule autour de l'axe de rotation 27, vers l'intérieur de la chambre 30 laissant libre l'espace central du connecteur 14, au travers duquel le contenu du récipient 31 peut être déchargé dans la chambre 30. Rien ne protège le contenu du récipient 31 lors de son transfert dans la chambre 30, au moment de son passage par un anneau sensible situé au niveau du joint 21 par lequel des agents contaminants risquent d'entrer dans l'espace de communication entre récipient 31 et chambre 30.

La figure 3 représente schématiquement une vue de coupe d'un exemple de deuxième type de récipient biopharmaceutique fretté avec trompe selon l'art antérieur.

La porte du récipient comprend une plaque métallique 23 surmoulée au niveau du centre du couvercle 22. Cette plaque métallique 23 est aimantée et est située du côté extérieur, donc à l'air libre et donc contaminée. Cette plaque métallique aimantée 23 plaque le connecteur 14 contre la porte de la chambre. Quand les portes, du récipient et de la chambre, sont déverrouillées, la porte de la chambre emmène avec elle la porte du récipient, la partie contaminée étant alors ainsi isolée de l'atmosphère stérile de la chambre.

Une trompe 19 de protection est fixée sur le connecteur 14 avec le rétrécissement 3 du récipient par frettage, c'est-à-dire en étant serrée sur le connecteur tout comme le rétrécissement 3 par une bague 20 de frettage. Ce deuxième type de récipient comprend de nombreuses pièces distinctes, dont la trompe 19 pièce rapportée par rapport au rétrécissement 3, et dont la bague 20 de frettage. La fabrication de ce deuxième type de récipient est relativement délicate et complexe.

La figure 4 représente schématiquement une vue de coupe d'un exemple d'un troisième type récipient biopharmaceutique soudé avec trompe.

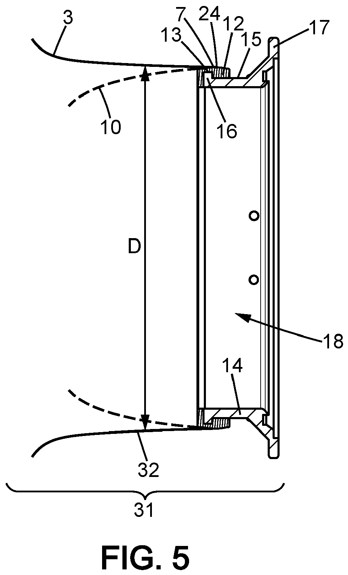

On peut imaginer d'améliorer le deuxième type de récipient en remplaçant le frettage du rétrécissement 3 et de la trompe 19 rapportée sur le connecteur 14 par un soudage à la fois du rétrécissement 3 et de la trompe 19 rapportée non pas directement sur le connecteur 14, mais sur un surmoulage 12 pour meilleure compatibilité de soudure. La trompe 19 rapportée pourrait être retroussée à l'intérieur du récipient 31. Le procédé de fabrication serait simplifié, car le soudage est plus simple que le frettage, et le nombre de pièces utilisées diminué, en raison de la suppression de la bague 20. Toutefois, ce troisième type de récipient possible, non encore connu de l'art antérieur, présenterait tout de même deux pièces distinctes, rétrécissement 3 et trompe rapportée 19, à souder ensemble soit directement sur le connecteur 14, soit par l'intermédiaire du surmoulage 12, d'où une certaine complexité restante que l'invention a choisi de simplifier, comme maintenant décrit et expliqué en liaison avec la figure 5.

La figure 5 représente schématiquement une vue de coupe d'un exemple d'un mode de réalisation d'un récipient biopharmaceutique soudé avec trompe selon l'invention. Le récipient 31 de la figure 5 peut être fixé de la même manière que le récipient 31 de la figure 1 sur la chambre 30 de la figure 2. Le diamètre D représente le diamètre de l'extrémité du rétrécissement 3 fixée sur le connecteur 14, au moment d'arriver sur le surmoulage 12. Ce diamètre D est plus grand que le diamètre d de l'extrémité ouverture du col 4.

Un récipient 31 comprend deux pièces principales qui sont une poche 1 souple mais non élastique et un connecteur 14 rigide, la poche 1 et le connecteur 14 étant tous deux cylindriques de révolution, la poche 1 étant fixée autour du connecteur 14 par l'intermédiaire d'un surmoulage 12 situé entre les deux. La poche 1, le connecteur 14 et le surmoulage 12, sont cylindriques à symétrie de révolution.

Le connecteur 14 comprend un rebord 17, une surface de fixation 15 présentant une protubérance 16. Au centre du connecteur 14 qui est annulaire se trouve l'espace de communication 18 situé entre récipient 31 et chambre non représentée sur cette figure 5 par souci de clarté mais représentée sur la figure 2.

Le surmoulage 12 comprend un pourtour 24 et un creux 13. Le surmoulage 12 est surmoulé de manière annulaire sur la surface de fixation 15 du connecteur 14, le creux 13 du surmoulage 12 s'emboîtant dans la protubérance 16 du connecteur 14.

La poche 1 comprend un fond 6, une paroi 32 cylindrique de révolution s'étendant le long d'un corps 2 et d'un rétrécissement 3. Dans la paroi 32 de la poche 1, un repli 7 est formé. De part et d'autre de ce repli 7 de la paroi 32 de la poche 1, se situent respectivement d'une part le rétrécissement 3 de la poche 1 et d'autre part la trompe 10 faisant partie intégrante de la poche 1 et constituant le prolongement de cette paroi 32 de la poche 1. Le repli 7, formé de la superposition des deux couches respectivement constituées par le rétrécissement 3 et la trompe intégrée 10, est soudé de manière annulaire sur le pourtour 24 du surmoulage 12, de manière à ce que le rétrécissement 3 soit soudé contre la trompe intégrée 10 elle-même soudée contre le pourtour 24 du surmoulage 12.

Il ne manque plus qu'un élément obturateur pour fermer l'espace de communication 18 pour que le récipient 31 soit finalisé. Soit le récipient 31 est rempli d'abord puis l'élément obturateur est mis en place ensuite, soit l'élément obturateur est mis en place d'abord, puis le récipient 31 est rempli ensuite, et enfin le fond 6 est soudé au corps 2 pour fermer la poche 1 et donc le récipient 31 au lieu d'avoir été préalablement soudé au corps 2.

Dans une alternative non représentée sur la figure 5, la poche 1 peut être en particulier une poche adaptée pour le transfert d'un fluide. Dans ce cas, un tuyau ayant à son extrémité un connecteur fluidique permettant une connexion aseptique traverse stérilement la paroi 32 de la poche 1. Ce tuyau pourra ainsi alors être inséré stérilement dans la chambre 30 pour former une ligne stérile entre d'une part une extrémité du tuyau, par laquelle peut être entré le contenu biopharmaceutique, et d'autre part la chambre 30 jusqu'à laquelle ce contenu biopharmaceutique peut être acheminé.

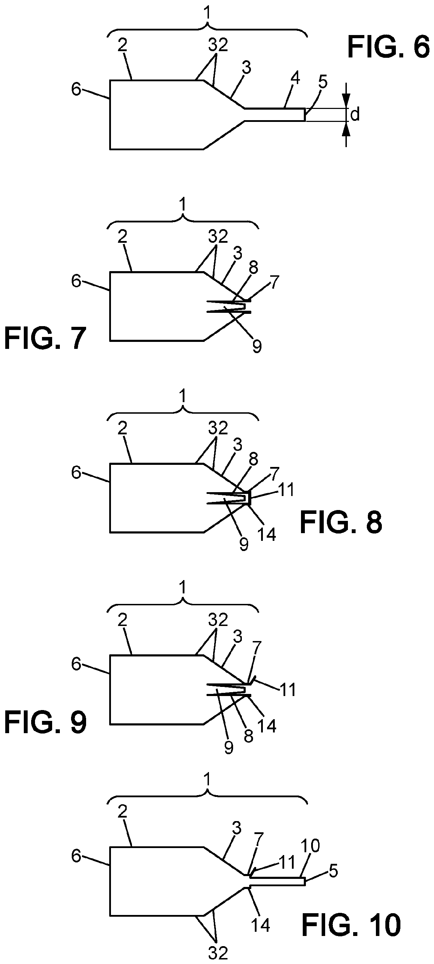

La figure 6 représente schématiquement un exemple d'une première étape d'un procédé de fabrication d'un mode de réalisation d'un récipient biopharmaceutique soudé avec trompe selon l'invention, ce récipient étant représenté de profil.

Une poche 1, sous forme de film plastique globalement cylindrique de révolution, est fabriquée dans une première étape. Cette poche 1 comprend une paroi 32, s'étendant entre le fond 6 de la poche 1 et l'ouverture 5 du col 4, en englobant le corps 2, le rétrécissement 3 et le col 4 de la poche 1. La poche 1 a une forme globale de bouteille, le diamètre d de l'ouverture 5 du col valant par exemple 110mm, voire 200mm. En revanche la paroi 32, incluant le corps 2, le rétrécissement 3 et le col 4, est d'un seul tenant. Le corps 2 et le col 4 ont une forme cylindrique de révolution, tandis que le rétrécissement 3 a une forme conique de révolution.

La figure 7 représente schématiquement un exemple d'une deuxième étape d'un procédé de fabrication d'un mode de réalisation d'un récipient biopharmaceutique soudé avec trompe selon l'invention, ce récipient étant représenté de profil.

Le col 4 est retroussé sur la moitié de sa longueur 8, conservant le bout 9 non retroussé, et formant un repli 7 entre la partie 8 et le rétrécissement 3.

La figure 8 représente schématiquement un exemple d'une troisième étape d'un procédé de fabrication d'un mode de réalisation d'un récipient biopharmaceutique soudé avec trompe selon l'invention, ce récipient étant représenté de profil.

Le repli 7 est soudé sur le pourtour du connecteur 14 au centre duquel est placée une porte 11 venant fermer le récipient 31. La soudure du repli 7 est réalisée par exemple autour de 120°C, de manière à faire fondre la paroi 32, mais loin du point de fusion du connecteur 14, lequel peut valoir par exemple environ 200°C. Au niveau du repli 7, le rétrécissement 3 est soudé contre la partie 8 de la paroi 32 elle-même soudée sur le connecteur 14, soit directement soit par l'intermédiaire d'un surmoulage non représenté sur la figure 8 par souci de clarté.

La figure 9 représente schématiquement un exemple d'une première étape d'un procédé d'utilisation d'un mode de réalisation d'un récipient biopharmaceutique soudé avec trompe selon l'invention, ce récipient étant représenté de profil.

Une fois la troisième étape de fabrication terminée au niveau de la figure 8, le contenu du récipient 31 ayant été ajouté juste avant la fermeture par la porte 11, le récipient 31 peut être utilisé. Il est également possible de ne souder le fond 6, qu'à la fin de cette troisième étape de fabrication terminée au niveau de la figure 8, une fois la porte 11 installée, et après avoir rempli le récipient 31 par son fond 6. Une fois le récipient 31 fixé contre une ouverture de la chambre non représentée par souci de clarté, la porte 11 est ouverte, rendant accessible, depuis l'intérieur de la chambre, le bout 9 de la trompe 10 intégrée. L'utilisateur ou le robot, situé dans la chambre n'a qu'à saisir le bout 9 et à le tirer vers l'intérieur de la chambre (vers la droite sur la figure 9), pour déployer cette trompe 10 intégrée à la paroi 32 de la poche 1.

La figure 10 représente schématiquement un exemple d'une deuxième étape d'un procédé d'utilisation d'un mode de réalisation d'un récipient biopharmaceutique soudé avec trompe selon l'invention, ce récipient étant représenté de profil.

La trompe 10 intégrée a été déployée. Le contenu du récipient 31, situé dans le corps 2, peut passer dans le rétrécissement 3, puis par la trompe 10 intégrée, traversant alors l'espace de communication (non représenté par souci de clarté) situé entre récipient 31 et chambre, pour sortir de la trompe 10 intégrée par l'ouverture 5 pour arriver à l'intérieur de la chambre.

Le procédé de fabrication, tel que représenté aux figures 6 à 8, sera notamment utilisé pour assembler une poche 1 dont la paroi 32 est en film plastique avec un connecteur 14 en polycarbonate (PC) ou acrylonitrile butadiène styrène (ABS) ou en polyvinyldene fluoride (PVDF) ou en polybutylene terephthalate (PBT). De tels matériaux pour le connecteur 14 seront rigides et pourront supporter les efforts de verrouillage contre la porte de la chambre ainsi que résister aux différents traitements associés à la stérilisation vapeur (à des températures inférieures à 125°C), gamma ou à l'oxyde d'éthylène.

Le film plastique de la paroi 32 de la poche 1 pourra être notamment du polyéthylène (PE), du polyéthylène haute densité (PEHD) ou du polyéthylène basse densité (PELD).

Le produit du surmoulage 12 sera alors par exemple du polyéthylène (PE), haute densité (PEHD) ou sous forme d'élastomère thermoplastique (TPE) ou pourra également être de l'élastomère thermoplastique ou du polypropylène ou en polyvinyldene fluoride (PVDF). Le surmoulage 12 ne va pas fondre à la montée en température lorsqu'il est monté sur le connecteur 14 lequel encaissera alors sa déformation.

La soudure du film plastique du repli 7 sur le surmoulage 12 résiste bien à la stérilisation aux rayons gamma ou à la stérilisation vapeur.

Pour réaliser la soudure, le film de la paroi 32 de la poche 1 et le surmoulage 12 commenceront à fondre quasiment en même temps donc dans des plages de température proches de l'ordre de 120°C (loin des températures de soudure des matériaux choisis pour le connecteur 14 valant plutôt de l'ordre de 200°C).

Les options suivantes peuvent notamment être envisagées, parmi lesquelles :
- le connecteur 14 est en polyvinyldene fluoride (PVDF) et le surmoulage 12 est en polyéthylène haute densité (PEHD) ou en élastomère thermoplastique (TPE) ou PBT ou PMMA (polyméthacrylate de méthyle),
- le connecteur 14 est en polycarbonate (PC) et le surmoulage 12 est en polyéthylène haute densité (PEHD) ou en élastomère thermoplastique (TPE) ou PBT ou PMMA (polyméthacrylate de méthyle),
- le connecteur 14 est en acrylonitrile butadiène styrène (ABS) et le surmoulage 12 est en polyéthylène haute densité (PEHD) ou en élastomère thermoplastique (TPE) ou PBT ou PMMA (polyméthacrylate de méthyle),
- le connecteur 14 est en polybutylene terephthalate (PBT) et le surmoulage 12 est en polyéthylène haute densité (PEHD) ou en polyvinyldene fluoride (PVDF) ou PBT (notamment pour souder une poche en copolyester) ou PMMA (polyméthacrylate de méthyle).

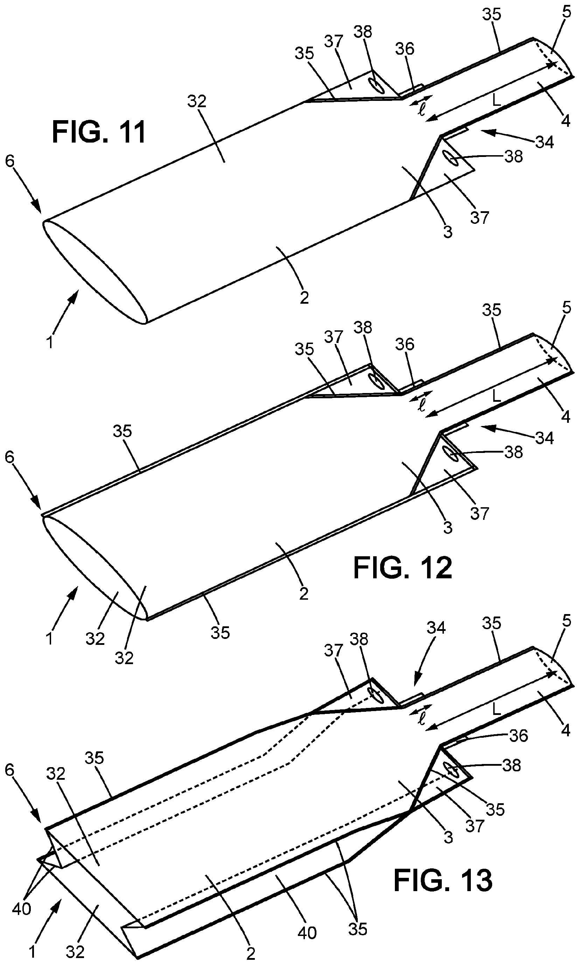

La figure 11 représente un premier exemple de type de procédé de fabrication d'une poche selon l'invention, par extrusion d'un film plastique.

La poche 1, sous forme de film plastique globalement cylindrique de révolution, comprend une paroi 32, s'étendant entre le fond 6 de la poche 1 et l'ouverture 5 du col 4, en englobant le corps 2, le rétrécissement 3 et le col 4 de la poche 1. La poche 1 a une forme globale de bouteille. La paroi 32, incluant le corps 2, le rétrécissement 3 et le col 4, est d'un seul tenant. Le corps 2 et le col 4 ont une forme cylindrique de révolution, tandis que le rétrécissement 3 a une forme conique de révolution.

La paroi 32 comprend principalement un film cylindrique extrudé constituant le corps 2, le rétrécissement 3 et le col 4 étant délimités par des soudures 35. A l'extérieur de l'espace intérieur de la poche 1, délimité par les soudures 35, se trouvent deux oreilles 37 percés de trous 38 permettant l'accrochage de la poche 1. Au niveau de l'extrémité du col 4 située du côté de l'extrémité de petite taille du rétrécissement 3, est réalisée une soudure supplémentaire 36, de longueur 1 valant environ 100mm, qui commence à l'extrémité du rétrécissement 3 et qui se termine au niveau de la marque visuelle 34 ; en fait, c'est la fin de la soudure supplémentaire 36 qui constitue une excroissance physique matérialisant la marque visuelle 34. Cette marque visuelle 34 sera utilisée pour retrousser le col 4 dans la poche 1 lors des étapes finales de la fabrication de la poche 1.

Le film plastique est souple mais non élastique. Ce film peut être constitué de couches de copolyester éthers, la poche 1 pouvant être stérilisée par vapeur et par rayons y (gamma). Ce film peut également être multicouche et être alors constitué d'une succession de couches suivantes : polyéthylène (PE) / polyacrylate (PA) / polyéthylène (PE), la poche 1 pouvant être stérilisée par rayons y.

La figure 12 représente un deuxième exemple de type de procédé de fabrication d'une poche selon l'invention, par soudage de deux feuilles de film plastique l'une sur l'autre.

Ce deuxième type de procédé de fabrication de poche se distingue du premier type de procédé de fabrication de poche précédemment décrit, également au niveau du résultat, c'est-à-dire de la poche obtenue. En effet, les soudures 35, situées de chaque côté du rétrécissement 3, se prolongent tout le long du corps 2.

Le film plastique est souple mais non élastique. Ce film peut être constitué d'une couche de polyéthylène haute densité (HDPE). La totalité ou au moins une partie de l'un des deux films soudés l'un sur l'autre, par exemple la moitié du corps 2 située le plus loin du rétrécissement 3, peut être constituée d'une couche de fibres interconnectées de polyéthylène haute densité (HDPE), la poche 1 pouvant alors être stérilisée par vapeur. Ce film peut également être constitué d'une couche de polyéthylène haute densité (HDPE). La totalité ou au moins une partie de l'un des deux films soudés l'un sur l'autre, par exemple la moitié du corps 2 située le plus loin du rétrécissement 3, peut être constituée d'une membrane en PES (polyéthylène sulfone), la poche 1 pouvant alors être stérilisée par vapeur. Cette membrane peut alternativement comprendre une membrane en polyéthylène sulfone (PES) et des flocons de fibres de polypropylènes (PP) revêtues de polyéthylène (PE).

La figure 13 représente un troisième exemple de type de procédé de fabrication d'une poche selon l'invention, par soudage de deux feuilles de film plastique l'une sur l'autre, avec soudage de deux replis latéraux entre les deux feuilles plastiques.

Ce troisième type de procédé de fabrication de poche se distingue du deuxième type de procédé de fabrication de poche précédemment décrit, également au niveau du résultat, c'est-à-dire de la poche obtenue. En effet, les soudures 35, situées de chaque côté du corps sont dédoublées, en raison de l'insertion, tout le long du corps 2, de replis latéraux 40 avantageusement en forme de V. Une fois la stérilisation réalisée, les soudures 35 de chaque côté de la poche 1 peuvent être ressoudées entre elles deux à deux, de manière à ce que les replis latéraux 40 soient alors entièrement à l'intérieur de la poche 1 et ne soient plus en contact direct avec le milieu extérieur à la poche 1.

Le film plastique est souple mais non élastique. Ce film peut être constitué de couches de copolyester éthers, la poche 1 pouvant être stérilisée par vapeur et par rayons y. Ce film peut également être multicouche et être constitué d'une succession de couches suivantes : polyéthylène (PE) / polyacrylate (PA) / polyéthylène (PE), la poche 1 pouvant être stérilisée par rayons y.

Ce film peut également être constitué d'une couche de polyéthylène haute densité (HDPE). La totalité ou au moins une partie de l'un des deux films soudés l'un sur l'autre, par exemple la moitié du corps 2 située le plus loin du rétrécissement 3, peut être constituée d'une membrane en PES, la poche 1 pouvant alors être stérilisée par vapeur au travers de cette membrane en PES. De préférence, les deux films sont constitués d'une couche de polyéthylène haute densité (HDPE). Les replis latéraux 40 sont alors constitués d'une membrane en PES, la poche 1 pouvant alors être stérilisée par vapeur au travers de cette membrane. Cette membrane peut alternativement comprendre une membrane en polyéthylène sulfone (PES) et des flocons de fibres de polypropylènes (PP) revêtues de polyéthylène (PE).

Ce film peut encore être constitué d'une couche de polyéthylène haute densité (HDPE). La totalité ou au moins une partie de l'un des deux films soudés l'un sur l'autre, par exemple la moitié du corps 2 située le plus loin du rétrécissement 3, peut être constituée d'une couche de fibres interconnectées de polyéthylène haute densité (HDPE), la poche 1 pouvant alors être stérilisée par vapeur, au travers de cette couche de fibres interconnectées de polyéthylène haute densité (HDPE). Les replis latéraux 40 sont constitués d'une couche de fibres interconnectées de polyéthylène haute densité (HDPE), la poche 1 pouvant alors être stérilisée par vapeur, au travers de cette couche de fibres interconnectées de polyéthylène haute densité (HDPE).

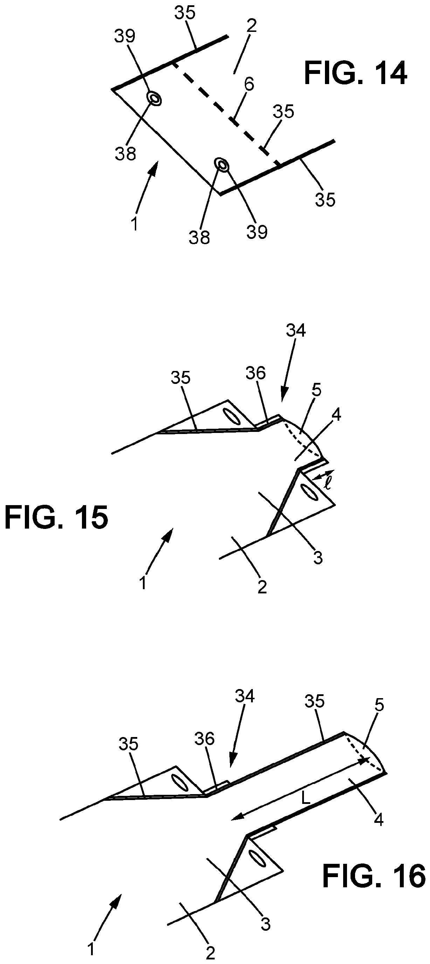

La figure 14 représente un exemple du fond d'une poche selon l'invention.

La poche 1, au niveau du corps 2, du côté du fond 6, présente deux soudures longitudinales 35 délimitant le corps 2, et une soudure 35 transversale appliquée après remplissage de la poche 1 par son contenu, de manière à constituer le fond 6 de la poche 1. Au-delà du fond 6, deux trous 38 sont réalisés pour pouvoir accrocher par le bas la poche 1. Des anneaux 39 colorés peuvent constituer un code couleur permettant de distinguer d'un simple coup d'œil le type de poche 1 concerné.

La figure 15 représente un exemple de poche selon l'invention, présentant une trompe courte.

La poche 1 représentée est une poche à col court 4 car ce col 4 ne prolonge le rétrécissement 3 que d'une longueur réduite, ici 1 valant par exemple environ 100mm. La soudure supplémentaire 34 s'étend sur toute la longueur du col court 4. La marque visuelle 34 est donc située à l'extrémité ouverte 5 du col court 4.

La figure 16 représente un autre exemple de poche selon l'invention, présentant une trompe longue.

La poche 1 représentée est une poche à col long 4 car ce col 4 prolonge le rétrécissement 3 d'une longueur importante, ici L valant par exemple environ 900mm. La soudure supplémentaire 34 ne s'étend que sur une partie de la longueur du col long 4, à savoir sur une longueur 1 valant par exemple 100mm. La marque visuelle 34 est donc située loin de l'extrémité ouverte 5 du col long 4.

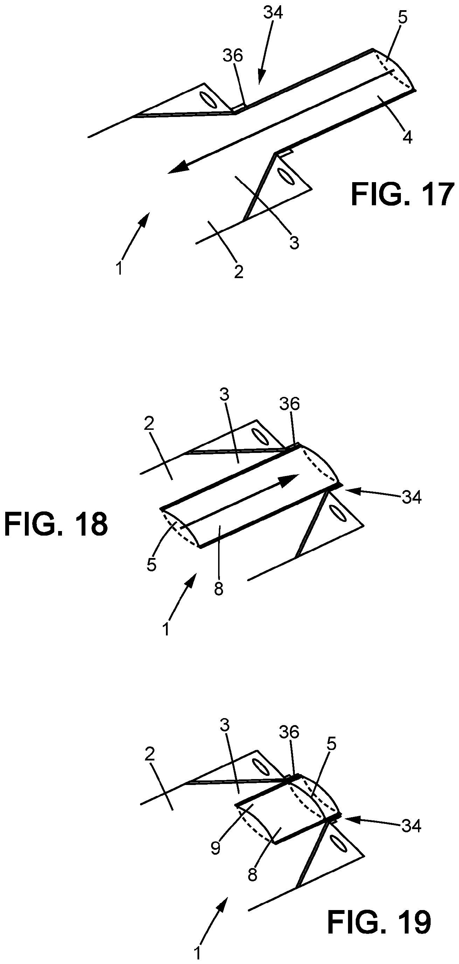

La figure 17 représente une étape du procédé de fabrication d'un exemple de poche selon l'invention, consistant essentiellement à retrousser le col dans la poche.

La flèche indique qu'une partie du col long 4 va être retroussée à l'intérieur de la poche 1, en fait même à l'intérieur du rétrécissement 3 et du reste du corps 2. Ce col long 4 va être retroussé dans la poche 1 jusqu'à atteindre le niveau de la marque visuelle 34.

La figure 18 représente une étape du procédé de fabrication d'un exemple de poche selon l'invention, consistant essentiellement à remettre le bout du col dans la partie retroussée elle-même située dans la poche.

La flèche indique qu'une partie du col long 4 retroussé à l'intérieur de la poche 1, en fait même à l'intérieur du rétrécissement 3 et du reste du corps 2, va maintenant être retroussée à l'intérieur de ce même col long 4 jusqu'à presque ressortir à l'extérieur de la poche 1, c'est-à-dire en fait jusqu'à affleurer au niveau de la marque visuelle 34.

La figure 19 représente un exemple de poche selon l'invention, prête à être fixée sur un connecteur.

La partie du col long 4 retroussée à l'intérieur de lui-même affleure au niveau de la marque visuelle 34. En particulier, le bout 9 du col 4 est prêt à être tirée vers l'extérieur en traversant le connecteur une fois que celui-ci aura été installé, pour se déployer à l'intérieur de la chambre à laquelle le connecteur aura été solidarisé.

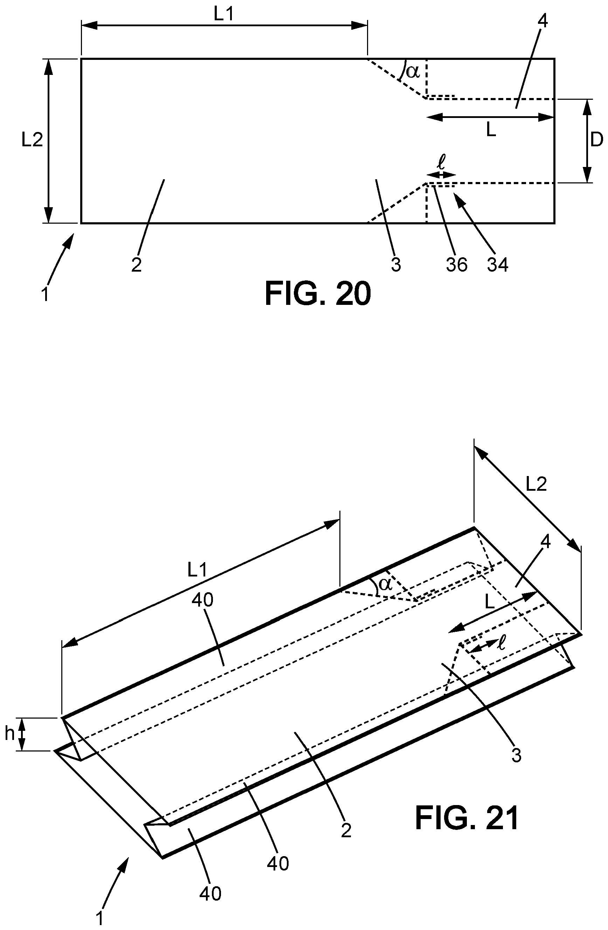

La figure 20 représente des exemples de dimensions d'une poche selon l'invention, correspondant aux premier et deuxième exemples de type de procédé de fabrication de la poche.

La longueur 1 de soudure supplémentaire 36 est comprise entre 80mm et 120mm, et vaut de préférence 100mm. La longueur L du col 4 est comprise entre 100mm et 900mm, de préférence comprise entre 300mm et 900mm, encore plus de préférence comprise entre 600mm et 900mm. Le diamètre D du col 4 est compris entre 300mm et 400mm, et vaut par exemple 340mm. La longueur L1 du corps 2 de la poche 1 est comprise entre 900mm et 1100mm, et vaut par exemple 1000mm. La largeur L2 de la poche 1 au niveau de son corps 2 est comprise entre 300mm et 600mm, de préférence comprise entre 400mm et 500mm. L'angle α du rétrécissement 3 vaut par exemple environ 45 degrés.

La figure 21 représente des exemples de dimensions d'une poche selon l'invention, correspondant au troisième exemple de type de procédé de fabrication de la poche.

Les dimensions sont similaires ou égales à celles de la figure 20. La hauteur h des replis latéraux 40 est comprise entre 20mm et 60mm, et vaut par exemple 40mm.

Bien entendu, la présente invention n'est pas limitée aux exemples et au mode de réalisation décrits et représentés, mais elle est susceptible de nombreuses variantes accessibles à l'homme de l'art.

## Revendications

1. Poche de récipient biopharmaceutique, comprenant :
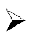 un corps (2) de poche (1) à fonction de contenant,
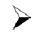 une partie de la poche (1) destinée à être fixée à un connecteur (14) du récipient (31),
**caractérisée en ce qu'**elle comprend aussi :
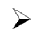 une trompe (10) flexible, qui est retroussée dans le corps (2) de la poche (1) et déployable à l'extérieur du corps (2) de la poche (1) au travers de la partie de la poche (1) destinée à être fixée à un connecteur (14) du récipient (31), qui est un col (4) de taille réduite prolongeant le corps (2) de la poche (1),
et **en ce que** :
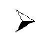 le col (4) comprend une marque visuelle (34) représentative de la position limite de retroussage du col (4) à l'intérieur du corps (2) de la poche (1),
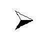 le col (4) comprend au moins une soudure longitudinale (35) délimitant le col (4) en s'étendant tout le long du col (4),
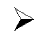 le col (4) comprend au moins une soudure supplémentaire (36) s'étendant le long d'une partie seulement de cette soudure longitudinale (35), l'extrémité de cette soudure supplémentaire (36), située du côté de l'ouverture (5) du col (4), constituant ladite marque visuelle (34).

2. Poche de récipient biopharmaceutique, comprenant :
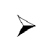 un corps (2) de poche (1) à fonction de contenant,
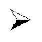 un col (4) de taille réduite, qui prolonge le corps (2) de la poche (1), qui est destinée à être une trompe (10) flexible de protection du transfert du contenu de la poche (1), et qui fait partie intégrante de la poche (1),
**caractérisée en ce que** :
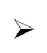 le col (4) comprend une marque visuelle (34) représentative de la position limite de retroussage du col (4) à l'intérieur du corps (2) de la poche (1).

3. Poche de récipient biopharmaceutique selon la revendication 2, **caractérisée en ce que** :
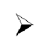 cette marque visuelle (34) est la fin d'une longueur (1) de soudure supplémentaire (36) le long de la poche (1), le début de cette longueur (1) de soudure supplémentaire (36) le long de la poche (1) coïncidant avec l'extrémité du col (4) de la poche (1) jouxtant le rétrécissement (3) de la poche (1).

4. Poche de récipient biopharmaceutique selon l'une quelconque des revendications précédentes, **caractérisée en ce qu'**elle comprend :
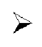 une partie de la poche (1) destinée à être fixée à un connecteur (14) du récipient (31),
**en ce que** :
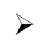 la trompe (10) flexible, est retroussée dans le corps (2) de la poche (1) et déployable à l'extérieur du corps (2) de la poche (1) au travers de la partie de la poche (1) destinée à être fixée à un connecteur (14) du récipient (31),
et **en ce que** :
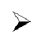 la partie de la poche (1) destinée à être fixée à un connecteur (14) du récipient (31) est un repli (7) de la paroi (32) de la poche (1),
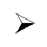 la paroi (32) de la poche (1) forme d'une part le corps (2) de la poche (1) d'un côté du repli (7) et d'autre part la trompe (10) de l'autre côté du repli (7).

5. Poche de récipient biopharmaceutique selon la revendication 4, **caractérisée en ce que** :
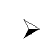 la trompe (10) n'est retroussée que sur une partie de sa longueur, avantageusement sur une partie comprise entre un tiers et deux tiers de sa longueur, encore plus avantageusement sur une partie valant environ la moitié de sa longueur,
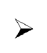 et/ou la trompe (10) présente une longueur qui est comprise entre 100mm et 900mm, de préférence comprise entre 300mm et 900mm, et encore plus de préférence comprise entre 600mm et 900mm.

6. Poche de récipient biopharmaceutique selon l'une quelconque des revendications précédentes, **caractérisée en ce que** :
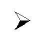 la poche (1) comprend un rétrécissement (3) progressif du corps (2) de la poche (1) avant la trompe (10) ou le col (4) de la poche (1).

7. Poche de récipient biopharmaceutique selon l'une quelconque des revendications précédentes, **caractérisée en ce que** :
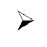 la trompe (10) ou le col (4) présente une section au moins 4 fois plus petite que la section du corps (2) de la poche (1), avantageusement au moins 10 fois plus petite.
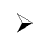 et/ou la trompe (10) ou le col (4) présente une longueur valant au moins le dixième de la longueur du corps (2) de la poche (1), avantageusement au moins le quart.

8. Récipient biopharmaceutique comprenant une poche de récipient biopharmaceutique selon l'une quelconque des revendications précédentes.

9. Récipient biopharmaceutique selon la revendication 8, **caractérisé en ce qu'**il comprend :
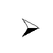 un connecteur (14) destiné à se fixer sur un connecteur de chambre (30),
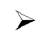 une partie de la poche (1), à fonction de contenant, étant fixée au connecteur (14),
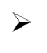 la trompe (10) flexible retroussée dans la poche (1) étant déployable à l'extérieur de la poche (1) au travers du connecteur (14),
et **en ce que** :
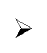 ladite partie de poche (1) fixée au connecteur (14) est un repli (7) de la paroi (32) de la poche (1),
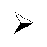 la paroi (32) de la poche (1) forme d'une part le corps (2) de la poche (1) d'un côté du repli (7) et d'autre part la trompe (10) de l'autre côté du repli (7).

10. Récipient biopharmaceutique selon la revendication 9, **caractérisé en ce que** :
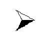 ladite partie de la poche (1) fixée au connecteur (14) est une partie de la poche (1) fixée autour du connecteur (14),
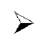 ou ladite partie de la poche (1) fixée au connecteur (14) est une partie de la poche (1) fixée sur le pourtour intérieur du connecteur (14).

11. Récipient biopharmaceutique selon l'une quelconque des revendications 9 à 10, **caractérisé en ce que** :
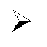 le repli (7) de la paroi (32) de la poche (1) comprend deux portions de paroi (32) de poche (1) repliées l'une sur l'autre, sans espace libre entre elles, ces deux portions étant avantageusement fixées l'une contre l'autre, ces deux portions étant plus avantageusement soudées l'une contre l'autre,
**en ce que** de préférence :
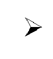 le repli (7) de la paroi (32) de la poche (1) est fixé sur le connecteur (14), un surmoulage (12) étant disposé entre le connecteur (14) et le repli (7), le repli (7) étant avantageusement soudé sur le surmoulage (12),
**en ce que** encore plus de préférence :
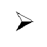 le surmoulage (12) comprend un creux (13) dont la forme intérieure épouse la forme extérieure d'une protubérance (16) de la paroi extérieure (24) du connecteur (14).

12. Récipient biopharmaceutique selon l'une quelconque des revendications précédentes, **caractérisé en ce que** :
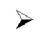 la poche (1) est à symétrie de révolution ou la partie de la poche (1) destinée à être soudée au connecteur (14) est à symétrie de révolution.

13. Récipient biopharmaceutique selon l'une quelconque des revendications précédentes, **caractérisé en ce que** :
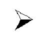 le connecteur (14) présente un diamètre (d) compris entre 80mm et 300mm, de préférence entre 100mm et 210mm ou bien le connecteur (14) présente une forme rectangulaire avec une longueur de diagonale comprise entre 100mm et 350mm.

14. Procédé d'utilisation d'un récipient biopharmaceutique selon l'une quelconque des revendications 9 à 13, **caractérisé en ce qu'**il comporte :
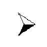 une étape de fixation du récipient (31) contre une ouverture (33) d'une chambre (30),
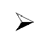 une étape de déploiement de la trompe (10) flexible hors du corps (2) de la poche (1) au travers du connecteur (14) et au travers de l'ouverture (33) de la chambre (30),
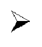 une étape de transfert du contenu du récipient (31) vers la chambre (30) au travers de la trompe (10) déployée,
et **en ce qu'**il comporte de préférence :
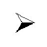 entre l'étape de fixation et l'étape de déploiement, une étape d'ouverture du connecteur (14) de la poche (1) et de la chambre (30), permettant une mise en communication des espaces intérieurs stériles de la poche (1) et de la chambre (30),
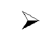 après l'étape de transfert, successivement une étape d'escamotage de la trompe (10) flexible, une étape de fermeture du connecteur (14) de la poche (1) et de la chambre (30), et une étape de déconnexion du récipient (31) et de la chambre (30).
